# EUROPEAN PATENT SPECIFICATION

(11) **EP 1 534 352 B1**
(45) Date of publication and mention of the grant of the patent: **10.09.2008**
(21) Application number: 03762631.4
(22) Date of filing: 04.07.2003
(51) Int. Cl.: A61L 27/18, A61L 27/54

(54) **IMPLANT FOR TRANSPORT AND RELEASE FOR PHARMACOLOGICALLY ACTIVE AGENTS AS WELL AS A PROCESS FOR PRODUCING THE SAME**
IMPLANTAT FÜR DEN TRANSPORT UND DIE FREIGABE VON PHARMAKOLOGISCH AKTIVEN SUBSTANZEN
IMPLANT PERMETTANT DE TRANSPORTER ET DE LIBERER DES AGENTS PHARMACOLOGIQUEMENT ACTIFS ET SON PROCEDE DE PRODUCTION

(30) Priority: 05.07.2002 DE 10230190
(43) Date of publication of application: 01.06.2005
(73) Proprietor: CeloNova BioSciences Germany GmbH, 89077 Ulm (DE)
(72) Inventor: DENK, Roman, 89197 Weidenstetten (DE)
(74) Representative: Teipel, Stephan
(86) International application number: PCT/EP2003/007197
(87) International publication number: WO 2004/004795

(56) References cited:
- EP-A- 1 179 353
- WO-A-02/41929
- IBIM S M ET AL: "Controlled macromolecule release from poly(phosphazene) matrices" JOURNAL OF CONTROLLED RELEASE, ELSEVIER SCIENCE PUBLISHERS B.V. AMSTERDAM, NL, vol. 40, no. 1, 1 June 1996 (1996-06-01), pages 31-39, XP004037349 ISSN: 0168-3659
- LEMMOUCHI Y ET AL: "BIODEGRADABLE POLYPHOSPHAZENES FOR DRUG DELIVERY" MACROMOLECULAR SYMPOSIA, WILEY VCH, WEINHEIM, DE, vol. 123, 1 September 1997 (1997-09-01), pages 103-112, XP000727295 ISSN: 1022-1360

## Description

The present invention relates to an implant for transport and release of pharmacologically active agents as well as a process for producing the same.

The most serious complications caused by artificial implants are considered to be the increased deposition of thrombocytes on their exogenous surface. Further, the behaviour *vis-à-vis* bacteria, macrophages and proteins depositing on the surfaces, plays an important role, since these depositions mainly lead to irritations and reactions of the immune system during the growing in of implants. This is shown macroscopically, e.g., in the occurrence of inflammation reactions. In case of cardiovasculary stents, this leads to an increased cytogenesis and an increased growth of intima, resulting in a renewed blockage of a vessel. In general, this phenomenon is called as restenosis. In other devices this phenomenon leads to the necessity of an additional treatment with e.g. antibiotically active agents.

By applying bis-(trifluoroethoxy)polyphosphazene for the coating of implants of all kinds and catheters and other medically used devices, said afore-mentioned side reactions and inflammation reactions can be substantially suppressed. Further, particularly the hemocompatibility and thrombogenity of such surfaces is significantly improved. This material primarily has a passive protective function in such a way that native proteins are reversibly resorbed on said surfaces. However, should there be, in spite of the excellent biocompatibility of this layer, an undesired cell growth, this kind of surface treatment fails to offer an additional possibility to suppress said growth.

It turned out that, particularly in vasculary and cardiovasculary stents, the excellent properties of this material are suited to significantly improve the hemocompatibility as well as the biocompatibility of a steel or nitinol surface *in vitro* and in a rabbit test. The incorporation of medicaments into said layer is also known to pharmacologically ensure the prevention of restenosis.

In addition, other forms of surface treatment are used to improve the surface and the compatibility, in particular to cardiovasculary stents. In this context, it is referred to attempts for improving the surface by means of coatings consisting, e.g., of SiC, TiN, gold or other metals or hard materials or organic molecules, such as polymethacrylate or proteinaceous components, or cellular components, such as phosphoryl choline. Polyfluorinated variants of a set of materials have been also tested and analyzed.

In the past these coatings have turned out to be only of limited activity. Recent developments contain, in addition to a layer consisting e.g. of a biologically degradable component, which may e.g. consist of polylactide, a pharmacologically active component, such as rapamycin, taxol or other pharmacologically active substances. Other materials are also suited as a coating for pharmacologically active agents, but the possible fields of application of these materials are limited by the physical chemical properties of these compounds and the galenic formulation of the pharmacologically active substances.

Because of the extremely limited amount of a suited material (about 1 cm² total stent surface and a layer thickness of at most about 5 µm) such agent/carrier combinations will release the active agent only for a period of some weeks. This is due to the degradation of the coating layer, in case of biologically degradable layers, and the release of the active substances, which can only be provided in a limited amount. After releasing the agents, it is likely that side reactions due to the degradation of the coating layer occur.

In the prior art a variety of materials is known and has been analyzed, which may be used for the production of implants and are also suited as coating layers for pharmacologically active substances in the afore-mentioned limits. For example, in WO 98/56312 an expandable shell of e-PTFE is used as an implant. Pharmacologically active substances can be incorporated into this shell. Other materials for this use are materials which are cited in EP-A-0 810 845, US-patent 4,883,699 and US-patent 4,991,691. Additional polymers known for this purpose are for example hydrolyzed polyacrylonitrile (US-patent 4,480,642), hydrophilic polyether (US-patent 4,798,876), polyurethanediacrylate (US-patent 4,424,395); in addition, numerous hydrogels are known and may be used for this purpose. Potentially suitable materials may be extended by polyvinylpyrrolidone (PVP)-polymers, polyvinylalcohole (PVA), p(polyethylene oxide)-polymers (PEO) and polyhydroxyethyl methacrylate p(HEMA). Moreover, there are documents which describe the use of standard materials, such as polyurethane, polyethylene and polypropylene, as possible materials. Mixtures of these materials are also known. Additional materials are known from EP-A-0 804 909, which are suited as a coating for pharmacologically active agents. The properties of these compounds are different and it is considered that each of these materials is more or less suited as a coating material for pharmacologically active agents. For example, PVA is highyl soluble in liquids and thus, guarantees an extremely rapid release of the incorporated agent. However, in case of cardiovasculary stents, a rapid release of the agent is not desired, but to achieve a continuous, linear or constant activity over a long period of time.

Polyphosphazenes are a known class of compounds, which have been tested in the production of implants and in the improvement of the surface properties of such implants. From the literature several examples of polyphosphazenes are known and analyzed, having a very good biocompatibility, in particular hemocompatibility (DE 19613048). This property is also maintained when increasing the degree of fluorination. Moreover, the elasticity of such compounds is improved by incorporating higher fluorinated and longer side chains. However, an improvement of the biological properties or a therapeutic activity is not achieved by incorporating said side chains.

Moreover, several materials are know, wherein the side chain may be replaced by a pharmacologically active substance. These materials are, for example, cis-platinum derivatives, and other agents which are released by the degradation of the polymer. Fluoro derivatives are also known and analyzed (Allcock et al., Phosphorous and Nitrogen Compounds, Academic Press, 1972); in particular the trifluoroethoxy variant is well characterized with respect to their physical chemical properties. In the same manner the perfluorinated variants of these materials are known and analyzed. Although all of these compounds contribute to the improvement of the compatibility of implants and other medical devices in the body, these compounds are not ideal, since they have no biological activity which continues for a long period of time or they are degraded. Further, substitution degrees of several percent, usually more than 5 %, as a minimum amount, are described in the literature to achieve the afore-mentioned effects (improvement of the elasticity). This is due to the branched main chain caused by a very strong tendency to hydrolysis and banching of the intermediates (polydichlorphosphazene). Thereby, only reduced substitution degrees may be achieved which are characterized by a relative high chlorine content (> 0,05% of the final stage).

The polymer compound poly[bis(trifluoroethoxy)phosphazene], as a volume material, shows a good antithrombogenic activity (cf. Tur, "Untersuchungen zur Thrombenresistenz von Poly[bis(trifluorethoxy)phosphazen]" und Holleman Wiberg, "Stickstoff-Zusammensetzungen des Phosphors", Lehrbuch der Anorganischen Chemie, 666-669, 91.-100. edition, Walter-de-Gruyter, 1985, and Tur, Vinogradova, "Entwicklungstendenzen bei polymeranalogen Umsetzungen von Polyphosphazen", Acta Polymerica 39, 424-429, Nr. 8, 1988), and perfluorinated polyphosphazenes are described in DE 196 13 048 as a material for coating artificial implants, in particular for the coating of heart valves. However, these materials as described so far do not exhibit the required mechanical properties for the use as a matrix material or volume material (i.e. as the structure forming material) in combination with the required release rate of a pharmacologically active agent.

Thus, the technical problem underlying the present invention is to provide an implant which enables the safe transport and/or sustained release of a pharmacologically active agent (i.e. constant release of the active agent) into the body over a sufficient period of time, in particular into a vessel, and which is suited to sufficiently support the location of action, e.g., the vessel, mechanically and pharmacologically during the healing.

The solution to the above object is achieved by the embodiments as characterized in the claims.

In particular, the present invention provides an implant comprising a homogenous and stable mixture of poly [bis(trifluoroethoxy) phosphazene and
(b) at least one pharmacologically active agent selected from tacrolimus or taxol incorporated into the matrix material.

Surprisingly, the physical properties of polyphosphazenes and the disadvantages associated therewith are positively changed by admixing at least one pharmacologically active agent. That is, a mixtures of a specific polyphosphazene polymer, as defined above, with at least one pharmacologically active agent exhibits mechanical properties which enable the use of such a material as a matrix material for transport and/or sustained release of pharmalogically active agents and for mechanically supporting the location of action, such as vessels, if the implant is a stent, in a patient.

In this context, it is emphasized that the mixture of the above polymer and the pharmacologically active agent is not used as a coating of an implant, but as a matrix or volume material which forms substantially the structure of the (whole) implant.

According to the present invention, the implant consists of (a) a polymer poly[bis(trifluoroethoxy)phosphazene (in the following PTFEP) as a matrix material, as defined herein, and (b) at least one pharmacologically active agent selected from tacrolimus or taxol incorporated into the matrix material, as defined below.

The terms "matrix material" and "volume material" as used herein relate to the structure forming material of the implant, for example a stent, which comprises the above polymer having the formula (I). Within the meaning of the present invention, a matrix material does not relate to a main constituent of a coating of an implant, but to the structure forming material itself.

The pharmacologically active agent may be incorporated in any form in the polymer PTFEP of the implant according to the present invention, for example, as a naked molecule or in nano- or micro-encapsulated form, so that the release of the agent may be regulated in a purposive manner. It is particularly preferred that the polymer PTFEP and the pharmacologically active agent form a homogenous and stable mixture to ensure a constant drug release profile over a long period of time.

The term "implant" encompasses any kind of suited implants, particulary implants which come into direct contact with tissue and/or body fluids of a patient. Examples of said implant are implants for e.g. breast, nose or ear, bone nails, bone screws, bone plates, artificial (urinary) bladder, artificial cartilage, dental implants, artificial bones for e.g. artificial hip or hip joints, artificial esophagus and artificial trachea; artificial (arterial and veinous) blood vessels; stents such as urological stents and cardiovascular stents; catheters such as urological catheters and cardiovascular catheters; cardiovascular grafts; emplastrums; dermatoplastics; seeds for the treatment of prostate hyperplasia; devices, e.g. in the gastrointestinal tract, in the prostata, in the urinary tract, or for the protection of neurons and neurofibers. Preferably the implant according to the present invention is in the form of a stent, in particular in the form of a cardiovasculary stent or an urological stent.

The polymer is poly[bis(trifluoroethoxy)phosphazene] (PTFEP), represented by the following formula:

-[N=P(OCH₂CF₃)₂]ₘ-

wherein m is 2 to ∞, preferably at least 50,000 and most preferably 50,000 to 60,000, based on the above repeating unit.

The pharmacologically active agent to be used in the implant is tacrolimus, or taxol (cf. R.T. Liggins, W.L. Hunter and H.M. Burt, Journal of Pharmaceutical Sciences, Vol. 86, No. 12, 1997). By using said pharmacologically active agents (alone or in a mixture), a homogenous and stable mixture in poly[bis(trifluoroethoxy)phosphazene], can be obtained.

It is desirable that the content of pharmacologically active agent(s) In the implant according to the present invention is as high as possible to e.g. prevent disorders caused by the implant such as restenosis, effectively. The weight ratio (mixing ratio) of PTFEP to the pharmacologically active agent(s) is preferably in a range of from about 50:1 to about 1:1, more preferably in a range of from about 10:1 to about 1:1, and most prefereably in a range of from about 5:1 to about 1:1. In this context, it is particularly preferred that PTFEP and the pharmacologically active agent(s) are miscible in each other and result in a homogenous and stable matrix material, and should preferably not result in a phase separation.

An implant in the form of a stent which is formed of poly[bis(trifluoroethoxy)phosphazene] (PTFEP) as a matrix material and (the) pharmacologically active substance(s) incorporated therein is particularly preferred.

The release of the pharmacologically active agents is influenced by the specific combination of the PTFEP and the pharmacologically active agent in a particular advantageous manner, so that the release rate of the pharmacologically active agent is significantly reduced and is suited to attain a constant release of the pharmacologically active agent over a sufficient period of time, such as several months, without damaging the surrounding cell tissue by a temporarily increased dosage.

The desired constant release rate of the pharmacologically active agent can be preferably achieved by a homogeneous and stable mixture of the polymer PTFEP and the at least one pharmacologically active agent. The "homogenous and stable mixture of the polymer PTFEP and the pharmacologically active agent" is achieved by adjusting suitable amounts of the polymer PTFEP and the pharmacologically active agent in such a way that no phase separation between the polymer PTFEP and the pharmacologically active agent will occur over a sufficient long period of time. An appropriate combination of a suitable amount of the polymer PTFEP and the suited pharmacologically active agent can be determined by a person skilled in the art.

Further, the elongation at break of the implant made of the PTFEP and a pharmacologically active agent is preferably at least 150 %. Thus, the mixing ratio of the polymer PTFEP and the pharmacologically active agent is preferably adjusted to obtain an elongation at break of at least 150 %.

The present invention further provides a process for producing an implant, as defined above, comprising the steps of:
(a) mixing the polymer PTFEP, as defined above, and at least one pharmacologically active agent, as defined above, and
(b) forming the mixture of step (a) Into a desired form.

The step of mixing the polymer PTFEP and at least one pharmacologically active agent is performed in the molten state or in solution, e.g. in an appropriate solvent. Appropriate solvents for this process may be selected from polar aprotic solvents such as esters (such as ethyl acetate, propyl acetate, butyl acetate, ethyl propionate, ethyl butyrate etc.), ketones (such as acetone, ethyl methyl ketone etc.), amides (such as dimethylformamide etc.), sulfoxides (such as DMSO etc.) and sulfones (such as sulfolane etc.). Ethyl acetate is particularly preferred.

Premixing of the substances in solution which are then crystallized and compressed, is also possible.

The step of forming the mixture into a desired from of an implant (the final form), preferably a stent, is preferably performed by high-compression (high-compacting) or by melt-extrusion.

The implant according to the present invention can be used in the blood stream, in the lower urinary tract, in bones, for treating nerves and nerve fibers, in the gastrointestinal tract, on the skin and under the skin (subcutaneous), as breast implant, in the bladder and the prostrate, in arteries or veins. Further, the implant according to the present invention can be used for dental implants or bone implants, for example bone nails, bone plates and bone screws.

The present invention further provides the use of a mixture containing a polymer and a pharmacologically active agent, as defined above, for the preparation of a pharmaceutical composition ("a galenic formulation") in the form of an implant for the treatment of disorders caused by implantation of said implant in a patient.

The subject matter of the present invention is further illustrated by way of the following examples without limitation thereto.

### Examples

The following examples 1 and 2, which relate to thick films, are given to simulate the implant according to the present invention, which is made of the above defined matrix material.

### Example 1

Polyzene^{®}-F, which is poly[bis(trifluoroethoxy)phosphazene] (PTFEP), and a pharmacologically active agent are mixed in a particular mass ratio in an appropriate amount of pure ethyl acetate. For test purposes said mixture is formed into a solution cast film as described in the test method for measuring the elongation at break as described below.

The elongation at break (%) of the above matrix material (containing polymer having the formula (I) and a pharmacologically active agent) is measured in the form of a solution cast film (formed from the specific die-cast as described below) in accordance with the method described in "Starannikova, L.E., et al. Vysokomolekulyarnye-Soedineniya, Ser. A & B, 36(11) (1994): 1,906", as follows:

A die-cast made of stainless steel having the dimension 7x1x50 mm was filled with a solution of the polymer having the formula (I), a pharmacologically active agent and ethyl acetate in various concentrations and was dried. The length of said film was determined and the film was enlongated up to the moment of break. The percentage as defined in the unit "%" relates to the elongation at the moment of break with respect to the initial length of the above film.

The evaluation results with respect to the properties of the resulting film are summarized in Tables 1 and 2.

**Table 1 (Taxol as active agent)**

| Composition of the mixture | Elongation at break | Quality of the film |
|---|---|---|
| no active agent, 20 mg Polyzene^{®}-F, 1ml ethyl acetate | 300 % | homogenous |
| 2 mg Taxol, 18 mg Polyzene^{®}-F, 1ml ethyl acetate | 220 % | homogenous |
| 5 mg Taxol, 15 mg Polyzene^{®}-F, 1ml ethyl acetate | 160 % | homogenous |

**Table 2 (Tacrolimus as active agent)**

| Composition of the mixture | Elongation at break | Quality of the film |
|---|---|---|
| no active agent, 20 mg Polyzene^{®}-F, 1ml ethyl acetate | 300 % | homogenous |
| 2 mg Tacrolimus, 18 mg Polyzene^{®}-F, 1 ml ethyl acetate | 212 % | homogenous |
| 5 mg Tacrolimus, 15 mg Polyzene^{®}-F, 1ml ethyl acetate | 190 % | homogenous |

### Example 2

The taxol containing poly[bis(trifluoroethoxy)phosphazene] (PTFEP) films as shown in Table 3 were prepared on a substrate from a solution containing taxol, poly[bis(trifluoroethoxy)phosphazene] and ethyl acetate by dip or spincoating. The film thickness was adjusted to about 0.3 to 1.0 µm. The resulting coating weight per surface unit is shown in Table 3.

In order to quantify the drug release properties of the samples, the increase of the drug concentration was measured by UV/vis-spectroscopy in a closed loop assembly in the course of one week.

**Table 3**

| PTFEP coating (µg/cm²) | taxol content (µg/cm²) | drug release |
|---|---|---|
| 175 | about 75 | initial burst effect, constant release after about 12h |
| 150 | about 50 | initial burst effect, constant release after about 12h |
| 125 | about 42 | initial burst effect, constant release after about 12h |
| 75 | about 32 | initial burst effect, constant release after about 12h |

As can be taken from the above test results, after an initial burst effect (lasting approximately 12 h), taxol is released in a linear relationship. The respective release rates thereof which can be calculated by a simple calibration method are in a range of from about 0.1 to about 3.3 µg/ml per week, depending on the initial loading.

## Claims

1. An implant comprising:
(a) a matrix material formed from a homogenous and stable mixture of poly[bis(trifluoroethoxy)phosphazene]
(b) at least one pharmacologically active agent selected from tacrolimus or taxol incorporated into the matrix material, wherein the implant resists restenosis and provides a constant rate of release of the at least one pharmacologically active agent over time.

2. The implant according to claim 1, wherein the implant is a stent.

3. The implant according to claim 1 or 2, wherein the weight ratio of polymer (I) to the pharmacologically active agent is in a range of from 10:1 to 1:1.

4. A process for producing an implant according to claims 1 to 3, comprising the steps of:
(a) mixing as homogenous and staste matrix material, poly[bis(trifluoroethoxy)phosphazene and at least one pharmaceutically active agent selected from the group consiting of tacrolimus or taxol and
(b) forming the mixture of step (a) into an implant.

5. The process according to claim 4, wherein the mixing in step (a) is performed in the molten state or in solution.

6. The process according to claim 4 or 5, wherein the step of forming the mixture into an implant in step (b) is performed by high-compression or by melt-extrusion.

7. The process according to any one of claims 4 to 6, wherein the implant is a stent.

8. Use of a mixture containing a polymer and a pharmacologically active agent as defined in any one of claims 1 to 3 for the preparation of a pharmaceutical composition in the form of an implant for the treatment of disorders caused by implantation of said implant in a patient.

## Patentansprüche

1. Implantat, umfassend:
(a) ein Matrixmaterial, gebildet aus einem homogenen und stabilen Gemisch aus Poly[bis(trifluorethoxy)phosphazen], und
(b) mindestens einen pharmakologischen Wirkstoff, ausgewählt aus Tacrolimus oder Taxol, eingeführt in das Matrixmaterial; wobei das Implantat gegen Restenose beständig ist und eine konstante Freisetzungsrate des mindestens einen pharmakologischen Wirkstoffes über die Zeit liefert.

2. Implantat nach Anspruch 1, wobei das Implantat ein Stent ist.

3. Implantat nach Anspruch 1 oder 2, wobei das Gewichtsverhältnis des Polymers (I) zu dem pharmakologischen Wirkstoff in einem Bereich von 10 : 1 bis 1 : 1 liegt.

4. Verfahren zur Herstellung eines Implantats nach den Ansprüchen 1 bis 3, umfassend die Schritte:
(a) des Mischens von Poly[bis(trifluorethoxy)phosphazen] als homogenes und stabiles Matrixmaterial und mindestens einem pharmakologischen Wirkstoff, ausgewählt aus der Gruppe, bestehend aus Tacrolimus oder Taxol, und
(b) des Formens des Gemisches aus Schritt (a) zu einem Implantat.

5. Verfahren nach Anspruch 4, wobei das Mischen in Schritt (a) in geschmolzenem Zustand oder in Lösung durchgeführt wird.

6. Verfahren nach Anspruch 4 oder 5, wobei der Schritt des Formens des Gemisches zu einem Implantat in Schritt (b) durch Hochkompression oder Schmelzextrusion durchgeführt wird.

7. Verfahren nach einem der Ansprüche 4 bis 6, wobei das Implantat ein Stent ist.

8. Verwendung eines Gemisches, enthaltend ein Polymer und einen pharmakologischen Wirkstoff, wie in einem der Ansprüche 1 bis 3 definiert, zur Herstellung einer pharmazeutischen Zusammensetzung in Form eines Implantats zur Behandlung von Erkrankungen, die durch Implantieren des Implantats bei einem Patienten verursacht werden.

## Revendications

1. Implant comprenant :
(a) une matière matricielle formée d'un mélange homogène et stable de poly[bis(trifluoroéthoxy)phosphazène]; et
(b) au moins un agent pharmacologiquement actif choisi parmi le tacrolimus et le taxol incorporé dans la matière matricielle, dans lequel l'implant résiste à la resténose et confère une vitesse de libération constante d'au moins l'agent pharmacologiquement actif en fonction du temps.

2. Implant suivant la revendication 1, dans lequel l'implant est un stent.

3. Implant suivant l'une ou l'autre des revendications 1 et 2, dans lequel le rapport en poids de polymère (I) à l'agent pharmacologiquement actif se situe dans la plage de 10/1 à 1/1.

4. Procédé de production d'un implant suivant l'une quelconque des revendications 1 à 3, comprenant les étapes de :
(a) mélange sous la forme d'une matière matricielle homogène et stable de poly[bis(trifluoroéthoxy)phosphazène] et d'au moins un agent pharmaceutiquement actif choisi dans le groupe comprenant le tacrolimus et le taxol, et
(b) formation du mélange de l'étape (a) en un implant.

5. Procédé suivant la revendication 4, dans lequel le mélange dans l'étape (a) est réalisé à l'état fondu ou en solution.

6. Procédé suivant l'une ou l'autre des revendications 4 et 5, dans lequel l'étape de formation du mélange en un implant dans l'étape (b) est réalisée par compression élevée ou par extraction de masse fondue.

7. Procédé suivant l'une quelconque des revendications 4 à 6, dans lequel l'implant est un stent.

8. Utilisation d'un mélange contenant un polymère et un agent pharmacologiquement actif tels que définis dans l'une quelconque des revendications 1 à 3 pour la préparation d'une composition pharmaceutique sous la forme d'un implant pour le traitement d'affections provoquées par l'implantation dudit implant chez un patient.
